# EUROPÄISCHE PATENTSCHRIFT

(11) **EP 0 696 572 B1**
(45) Veröffentlichungstag und Bekanntmachung des Hinweises auf die Patenterteilung: **01.10.1997**
(21) Anmeldenummer: 95111954.4
(22) Anmeldetag: 29.07.1995
(51) Int. Cl.: C07C 209/16, C07C 209/18

(54) **Verfahren zur Herstellung von Aminen**
Process for the preparation of amines
Procédé pour la préparation d'amines

(30) Priorität: 08.08.1994 DE 4428004
(43) Veröffentlichungstag der Anmeldung: 14.02.1996
(73) Patentinhaber: BASF AKTIENGESELLSCHAFT, 67056 Ludwigshafen (DE)
(72) Erfinder: Reif, Wolfgang, Dr., D-67227 Frankenthal (DE); Franz, Lothar, Dr., D-67112 Mutterstadt (DE); Stops, Peter, D-67122 Altrip (DE); Menger, Volkmar, Dr., D-67434 Neustadt (DE); Winderl, Siegfried, Dr., D-69118 Heidelberg (DE); Becker, Rainer, Dr., D-67098 Bad Dürkheim (DE); Kummer, Rudolf, Dr., D-67227 Frankenthal (DE)

(56) Entgegenhaltungen:
- EP-A- 0 382 049
- EP-A- 0 514 692

## Beschreibung

Die vorliegende Erfindung betrifft ein Verfahren zur katalytischen Aminierung von Alkoholen mit Stickstoffverbindungen und Wasserstoff in Gegenwart eines Zirkon-, Kupfer-, Nickelkatalysators bei erhöhten Temperaturen und Drücken mit Zirkon-, Kupfer-, Nickelkatalysatoren, deren aktive Masse sauerstoffhaltige Verbindungen des Molybdäns, enthält.

Aus DE-A-19 53 263 ist es bekannt, Amine durch die hydrierende Aminierung der entsprechenden Alkohole an Cobalt, Nickel und Kupfer enthaltenden Katalysatoren herzustellen. Als Trägermaterial wird in diesen Katalysatoren Aluminium oder Siliciumdioxid verwendet. Mit diesen Katalysatoren lassen sich bei hohen Temperaturen und Drücken gute Umsätze erzielen. Wird bei tieferen Temperaturen und Drücken gearbeitet, geht der Umsatz und die Selektivität stark zurück.

Aus der EP-A-254 335 sind Ni-Co-Ru-Katalysatoren auf Aluminiumoxid- oder Siliciumdioxid-Trägern, welche zusätzlich noch Halogenide in ihrer aktiven Masse enthalten, zur hydrierenden Aminierung von Alkoholen bekannt. Mit diesen Katalysatoren werden bei 200°C und 55 bar nur Umsätze von maximal 61 % erzielt.

Aus der US-A-4 151 204 sind Katalysatoren zur Herstellung von Aminoalkoholen bekannt, die aus einem Metall wie Cobalt, Nickel oder Kupfer, bevorzugt aus Nickel oder Cobalt bestehen, und gegebenenfalls mit geringen Mengen an Zirkonium dotiert sind, wobei das Zirkonium bezüglich des Nickels oder Cobalts in einem Molverhältnis von 0,005 : 1 bis 0,2 : 1 zugesetzt wird. Höhere Zirkoniumgehalt führen zu Nebenreaktionen wie der Zersetzung der Produkte.

Aus der EP-A-382 049 sind Katalysatoren und Verfahren zur hydrierenden Aminierung von Alkoholen bekannt. Diese Katalysatoren, deren aktive Masse sauerstoffhaltige Zirkon-, Kupfer-, Cobalt- und Nickelverbindungen enthält, zeichnen sich zwar durch eine gute Aktivität und Selektivität aus, besitzen allerdings verbesserungswürdige Standzeiten.

Der vorliegenden Erfindung lag daher die Aufgabe zugrunde, den zuvor genannten Nachteilen abzuhelfen.

Demgemäß wurde ein neues und verbessertes Verfahren zur Herstellung von Aminen aus primären oder sekundären Alkoholen und Stickstoffverbindungen, ausgewählt aus der Gruppe Ammoniak, primäre und sekundäre Amine, bei Temperaturen von 80 bis 250°C und Drücken von 1 bis 400 bar mit Wasserstoff in Gegenwart eines Zirkon-, Kupfer-, Nickelkatalysators gefunden, welches dadurch gekennzeichnet ist, daß die katalytisch aktive Masse 20 bis 85 Gew.-% sauerstoffhaltige Zirkon-Verbindungen, berechnet als ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃ und 0 bis 10 Gew.-% der sauerstoffhaltigen Verbindungen von Aluminium und/oder Mangan, berechnet als Al₂O₃ bzw. MnO₂ enthält. Bevorzugt ist die Herstellung von Aminen der allgemeinen Formel I 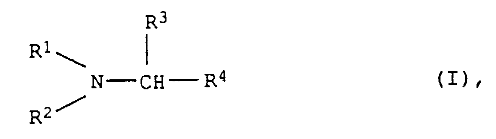 in der
- R¹ und R²: Wasserstoff, C₁- bis C₂₀-Alkyl, C₃- bis C₁₂-Cycloalkyl, Aryl, C₇- bis C₂₀-Aralkyl und C₇- bis C₂₀-Alkylaryl oder gemeinsam (CH₂)₁-X-(CH₂)ₘ,
- R³ und R⁴: Wasserstoff, C₁- bis C₂₀₀-Alkyl, C₃- bis C₁₂-Cycloalkyl, C₁- bis C₂₀-Hydroxyalkyl, durch Amino und/oder Hydroxy substituiertes C₁- bis C₂₀-Alkyl, C₂- bis C₃₀-Alkoxyalkyl, R⁵-(OCR⁶R⁷CR⁸R⁹)ₙ-(OCR⁶R⁷), Aryl, C₇- bis C₂₀-Aralkyl, C₇- bis C₂₀-Alkylaryl, (R⁵)₂N-(CH₂)_{q} und Y-(CH₂)ₘ-NR⁵-(CH₂)_{q} oder gemeinsam (CH₂)₁-X-(CH₂)ₘ oder
- R² und R⁴: gemeinsam (CH₂)₁-X-(CH₂)ₘ,
- R⁵: Wasserstoff, C₁- bis C₄-Alkyl, C₁₂- bis C₄₀-Alkylphenyl,
- R⁶,R⁷,R⁸,R⁹: Wasserstoff, Methyl und Ethyl,
- R¹⁰: Wasserstoff, C₁-C₄-Alkyl,
- X: CH₂, Sauerstoff oder N-R⁶,
- Y: N(R⁵)₂, Hydroxy, C₂- bis C₂₀-Alkylaminoalkyl oder C₃- bis C₂₀-Dialkylaminoalkyl,
- n: eine ganze Zahl von 1 bis 30,
- l: eine ganze Zahl von 2 bis 4,
- m, q: eine ganze Zahl von 1 bis 4
bedeuten, aus primären oder sekundären Alkoholen der allgemeinen Formel II

R⁴ - CHR³ - OH (II),

und Stickstoffverbindungen der allgemeinen Formel III 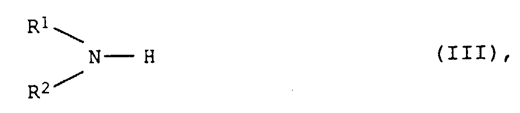 in der R¹, R² sowie R³ und R⁴ die oben genannten Bedeutungen haben.

Als Alkohole eignen sich praktisch alle primären und sekundären aliphatische Alkohole. Die aliphatischen Alkohole können geradkettig, verzweigt oder cyclisiert sein. Sekundäre Alkohole werden ebenso aminiert wie primäre Alkohole. Hinsichtlich der Kohlenstoffzahl der aminierbaren Alkohole sind bislang keine Beschränkungen bekannt. Die Alkohole können ferner Substituenten tragen, welche sich unter den Bedingungen der hydrierenden Aminierung inert verhalten, beispielsweise Alkoxy- oder Alkylenoxygruppen. Sollen mehrbasische Alkohole aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Alkohole aminiert:

Methanol, Ethanol, n-Propanol, Isopropanol, n-Butanol, iso-Butanol, n-Pentanol, n-Hexanol, 2-Ethylhexanol, Tridecanol, Stearylalkohol, Palmitylalkohol, Cyclopentanol, Cyclohexanol, Ethanolamin, n-Propanolamin, Isopropanolamin, n-Pentanolamin, n-Hexanolamin, Diethanolamin, N-Alkyldiethanolamine, Diisopropanolamin, Ethylenglykol, Propylenglykol, Butandiol, Pentandiol, Hexandiol, 4,4'-Bishydroxycyclohexylpropan-(2,2), Methoxyethanol, Propoxyethanol, Butoxyethanol, Polyisobutylalkohole, Polypropylalkohole, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Als Aminierungsmittel bei der hydrierenden Aminierung von Alkoholen können sowohl Ammoniak als auch primäre oder sekundäre, aliphatische oder cycloaliphatische Amine eingesetzt werden.

Bei Verwendung von Ammoniak als Aminierungsmittel werden die alkoholischen Hydroxylgruppen zunächst in freie Aminogruppen (-NH₂) umgewandelt. Die so gebildeten primären Amine können mit weiterem Alkohol zu den entsprechenden sekundären Aminen und diese wiederum mit weiterem Alkohol zu den entsprechenden, symmetrischen tertiären Aminen reagieren. Je nach Zusammensetzung des Reaktionsansatzes und je nach den angewandten Reaktionsbedingungen - Druck, Temperatur, Reaktionszeit - lassen sich auf diese Weise je nach Wunsch bevorzugt primäre, sekundäre oder tertiäre Amine darstellen.

Aus mehrbasischen Alkoholen lassen sich auf diese Weise durch intramolekulare hydrierende Aminierung cyclische Amine wie Pyrrolidine, Piperidine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Bevorzugt werden beispielsweise die folgenden Mono- und Dialkylamine als Aminierungsmittel verwendet: Methylamin, Dimethylamin, Ethylamin, Diethylamin, Propylamin, Diisopropylamin, Butylamin, Pentylamin, Hexylamin und Cyclohexylamin.

Das Aminierungsmittel kann bezüglich der zu aminierenden alkoholischen Hydroxylgruppe in stöchiometrischer Menge eingesetzt werden. Bevorzugt wird jedoch mit einem Überschuß an Aminierungsmittel gearbeitet und zwar im allgemeinen mit einem mehr als 5molaren Überschuß pro Mol zu aminierender alkoholischer Hydroxylgruppe. Speziell Ammoniak wird im allgemeinen mit einem 5 bis 250-fachen, bevorzugt 10 bis 100-fachen, insbesondere 25 bis 80-fachen molaren Überschuß pro Mol umzusetzender alkoholischer Hydroxylgruppen eingesetzt. Höhere Überschüsse sowohl an Ammoniak als auch an primären oder sekundären Aminen sind möglich.

Der Wasserstoff wird der Reaktion im allgemeinen in einer Menge von 5 bis 400 Nl, bevorzugt in einer Menge von 50 bis 200 Nl pro Mol Alkoholkomponente zugeführt.

Die Umsetzung erfolgt im allgemeinen ohne zusätzliches Lösungsmittel. Bei der Umsetzung hochmolekularer hochviskoser oder bei Raumtemperatur fester Ausgangsverbindungen oder Produkte kann es vorteilhaft sein ein unter den Reaktionsbedingungen inertes Lösungsmittel, wie Tetrahydrofuran, Dioxan, N-Methylpyrrolidon oder Ethylenglykoldimethylether mitzuverwenden.

Üblicherweise arbeitet man bei der Umsetzung bei Temperaturen von 80 bis 200°C, bevorzugt 120 bis 230°C, besonders bevorzugt 150 bis 220°C. Im allgemeinen wird die Reaktion bei einem Druck von 1 bis 400 bar ausgeführt. Bevorzugt werden jedoch Drücke von 10 bis 250 bar, insbesondere von 30 bis 200 bar angewandt.

Die Anwendung höherer Temperaturen und eines höheren Gesamtdruckes ist möglich. Der Gesamtdruck im Reaktionsgefäß, welches sich aus der Summe der Partialdrücke des Aminierungsmittels, der Alkoholkomponente und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck eingestellt.

Es kann für die Selektivität des vorliegenden Verfahrens vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Praktisch geht man bei der Durchführung im allgemeinen so vor, daß man dem Katalysator, der sich üblicherweise in einem bevorzugt von außen beheizten Festbettreaktor befindet, bei der gewünschten Reaktionstemperatur und dem gewünschten Druck den Alkohol und das Aminierungsmittel simultan zuführt. Dabei belastet man den Katalysator im allgemeinen mit 0,02 bis 3, bevorzugt 0,05 bis 2 und besonders bevorzugt mit 0,1 bis 1,6 l Alkohol pro Liter Katalysator und Stunde. Hierbei ist es zweckmäßig, die Reaktanten bereits vor der Zuführung in das Reaktionsgefäß zu erwärmen und zwar bevorzugt auf die Reaktionstemperatur.

Der Reaktor kann sowohl in der Sumpf- als auch in der Rieselfahrweise betrieben werden, d.h. man kann die Reaktanten sowohl von unten nach oben als auch von oben nach unten durch den Reaktor leiten. Es versteht sich von selbst, daß sich das Verfahren sowohl diskontinuierlich als auch kontinuierlich durchführen läßt. In beiden Fällen kann das überschüssige Aminierungsmittel zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Aus dem Reaktionsaustrag werden nachdem dieser zweckmäßigerweise entspannt worden ist, das überschüssige Aminierungsmittel und der Wasserstoff entfernt und die erhaltenen aminierten Produkte durch Destillation, Flüssigextraktion oder Kristallisation gereinigt. Das überschüssige Aminierungsmittel und der Wasserstoff werden vorteilhaft wieder in die Reaktionszone zurückgeführt. Das gleiche gilt für die eventuell nicht oder nicht vollständig umgesetzte Alkoholkomponente.

Das im Zuge der Umsetzung gebildete Reaktionswasser wirkt sich im allgemeinen auf den Umsetzungsgrad, die Reaktionsgeschwindigkeit, Die Selektivität und die Katalysatorstandzeit nicht störend aus und wird deshalb zweckmäßigerweise erst bei der destillativen Aufarbeitung des Reaktionsproduktes aus diesem entfernt.

Im allgemeinen werden die erfindungsgemäßen Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, daß man die katalytisch aktive, zu Pulver vermahlene Masse in das Reaktionsgefäß einbringt oder, daß man die katalytisch aktive Masse nach Mahlung, Vermischung mit Formhilfsmitteln, Formung und Temperung als Katalysatorformkörper - beispielsweise als Kugeln, Zylinder, Ringe, Spiralen - im Reaktor anordnet.

Die katalytisch aktive Masse der erfindungsgemäßen Katalysatoren enthält neben sauerstoffhaltigen Verbindungen des Zirkoniums, sauerstoffhaltige Verbindungen des Nickels, Kupfers und Molybdäns.

Da sich die Konzentrationsangaben jeweils - falls nicht anders angegeben - auf die katalytisch aktive Masse des Katalysators beziehen, wird die katalytisch aktive Masse des Katalysators im folgenden als die Summe der Massen der katalytisch aktiven Bestandteile Zirkonium, Nickel, Kupfer und Molybdän im Katalysator, jeweils berechnet als ZrO₂, NiO, CuO bzw. MoO₃, nach dessen letzter Wärmebehandlung und ver dessen Reduktion mit Wasserstoff, definiert.

Im allgemeinen beträgt der Zirkoniumoxidgehalt der erfindungsgemäßen Katalysatoren zwischen 20 und 85, bevorzugt 70 bis 80 Gew.-%.

Die anderen Komponenten Nickel und Kupfer sind im allgemeinen insgesamt in Mengen von 15 bis 80, bevorzugt 15 bis 60, insbesondere 15 bis 50 Gew.-% und Molybdän in Mengen von 0,1 bis 5 Gew.-%, bevorzugt 0,5 bis 3,5 Gew.-% in der katalytisch aktiven Masse enthalten.

Bevorzugte Katalysatoren enthalten in ihrer katalytisch aktiven Masse 20 bis 85 Gew.-%, bevorzugt 25 bis 60 Gew.-% sauerstoffhaltige Zirkoniumverbindungen, 1 bis 30 Gew.-%, bevorzugt 10 bis 25 Gew.-% sauerstoffhaltige Kupferverbindungen, 30 bis 70 Gew.-%, bevorzugt 40 bis 70 Gew.-%, besonders bevorzugt 45 bis 60 Gew.-% sauerstoffhaltige Verbindungen des Nickels, 0,1 bis 5 Gew.-%, bevorzugt 0,5 bis 3,5 Gew.-% sauerstoffhaltige Verbindungen des Molybdäns und 0 bis 10 Gew.-% sauerstoffhaltige Verbindungen von Aluminium und/oder Mangan.

Zur Herstellung der Vollkatalysatoren sind verschiedenerlei Verfahrensweisen möglich. Sie sind beispielsweise durch Anteigen pulvriger Mischungen der Hydroxide, Carbonate, Oxide und/oder anderer Salze der Komponenten Zirkonium, Nickel und Kupfer mit Wasser und nachfolgendes Extrudieren und Tempern der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Nickel- und Kupferkomponenten aus einer diese Elemente enthaltenden, wäßrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wäßrigen Zirkoniumsalzlösungen mittels Mineralbasen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wäßrige Salzlösung in der Wärme und unter Rühren so lange mit einer wäßrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch - da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, daß bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Erfindungsgemäße Katalysatoren mit besonders vorteilhaften Eigenschaften sind dadurch erhältlich, daß man einen Teil der Zirkoniumkomponente des Katalysators, zweckmäßigerweise aus einer wäßrigen Zirkoniumsalzlösung, separat in einer Fällungsapparatur durch Zugabe wäßriger Mineralbasen fällt. Auf das so erhaltene, vorzugsweise frisch gefällte Zirkoniumoxid-Hydrat, kann dann der restliche Teil der Zirkoniumkomponente des Katalysators zusammen mit den anderen katalytisch aktiven Komponenten in einer Mischfällung gefällt werden, wie oben beschrieben wurde. Dabei erweist es sich in der Regel als besonders zweckmäßig 10 bis 80 Gew.-%, vorzugsweise 30 bis 70 Gew.-% und insbesondere 40 bis 60 Gew.-% der Gesamtzirkoniummenge der katalytisch aktiven Masse vorzufällen.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der genannten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200°C, vorzugsweise bei 100 bis 150°C, getrocknet und danach calciniert. Die Calcinierung wird im allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, daß man ihn durch Vermahlen auf eine bestimmte Korngröße einstellt oder daß man ihn nach seiner Vermahlung mit Formhilfsmitteln wie Graphit oder Stearinsäure vermischt, mittels einer Tablettenpresse zu Formlingen verpreßt und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren zur hydrierenden Aminierung von Alkoholen werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200 bis 300°C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so daß diese gemeinsam mit den verschiedenartigen Sauerstoffverbindungen in der aktiven Form das Katalysators vorliegen.

Die Substituenten R¹, R², R³, R⁴, R⁵, R⁶, R⁷, R⁸, R⁹, R¹⁰ und die Indizes l, m und n in den Verbindungen I, II und III haben unabhängig voneinander folgende Bedeutungen:
R¹, R², R³, R⁴, R^{5,} R⁶, R⁷, R⁸, R⁹ R¹⁰
- Wasserstoff,
R³, R⁴
- C₁- bis C₂₀₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀- bis C₂₀₀-Alkyl wie Polybutyl, Polyisobutyl, Polypropyl, Polyisopropyl und Polyethyl, besonders bevorzugt Polybutyl und Polyisobutyl,
- R¹ und R² oder R³ und R⁴ oder R² und R⁴ gemeinsam eine -(CH₂)ₗ-X-(CH₂)ₘ-Gruppe,
R¹, R², R³, R⁴
- C₃- bis C₁₂-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
R¹, R²
- C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec-Butyl und tert.-Butyl,
R³, R⁴
- C₁- bis C₂₀-Hydroxyalkyl, bevorzugt C₁- bis C₈-Hydroxyalkyl, besonders bevorzugt C₁- bis C₄-Hydroxyalkyl wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methyl-ethyl,
- durch Amino und Hydroxy substituiertes C₁- bis C₂₀-Alkyl, bevorzugt durch Amino und/oder Hydroxy substituiertes C₁- bis C₈-Alkyl, besonders bevorzugt durch Amino und/oder Hydroxy substituiertes C₁- bis C₄-Alkyl wie N-(Hydroxyethyl)aminoethyl und N-(Aminoethyl)aminoethyl,
- C₂- bis C₃₀-Alkoxyalkyl, bevorzugt C₂- bis C₂₀-Alkoxyalkyl, besonders bevorzugt C₂- bis C₈-Alkoxyalkyl wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxy-ethyl, besonders bevorzugt C₂- bis C₄-Alkoxyalkyl wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl,
- R⁵-(OCR⁶R⁷CR⁸R⁹)ₙ-(OCR⁶R⁷), bevorzugt R⁵-(OCHR⁷CHR⁹)ₙ-(OCR⁶R⁷), besonders bevorzugt R⁵-(OCH₂CHR⁹)ₙ-(OCR⁶R⁷),
- (R⁵)₂N-(CH₂)_{q},
- Y-(CH₂)ₘ-NR⁵-(CH₂)_{q},
R⁵, R¹⁰
- C₁- bis C₄-Alkyl wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
R⁶, R⁷, R⁸, R⁹
- Methyl und Ethyl, bevorzugt Methyl,
X
- CH₂,
- Sauerstoff,
- N-R⁶,
Y
- N(R⁵)₂,
- Hydroxy,
- C₂- bis C₂₀-Alkylaminoalkyl, bevorzugt C₂- bis C₁₆-Alkylaminoalkyl wie Methylaminomethyl, Methylaminoethyl, Ethylaminomethyl, Ethylaminoethyl und iso-Propylaminoalkyl,
- C₃- bis C₂₀-Dialkylaminoethyl, bevorzugt C₃- bis C₁₆-Dialkylaminoalkyl wie Dimethylaminomethyl, Dimethylaminoethyl, Dialkylaminoethyl, Di-n-propylaminoethyl und Di-iso-propylaminoethyl,
l
- eine ganze Zahl von 2 bis 4 wie 2, 3 und 4, bevorzugt 2 und 3, besonders bevorzugt 2,
m, q
- eine ganze Zahl von 1 bis 4 wie 1, 2, 3 und 4, bevorzugt 2, 3 und 4, besonders bevorzugt 2 und 3,
R⁵
- C₁₂- bis C₄₀-Alkylphenyl, bevorzugt C₁₄- bis C₄₀-Alkylphenyl wie 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,
n
- eine ganze Zahl von 1 bis 10, bevorzugt eine ganze Zahl von 1 bis 8 wie 1, 2, 3, 4, 5, 6, 7 oder 8, besonders bevorzugt eine ganze Zahl von 1 bis 6 wie 1, 2, 3, 4, 5 oder 6.

Die erfindungsgemäß erhältlichen Amine eignen sich u.a. als Zwischenprodukte bei der Herstellung von Kraftstoffadditiven (US-A-3 275 554; DE-A-21 25 039 und DE-A-36 11 230), Tensiden, Arznei- und Pflanzenschutzmitteln sowie von Vulkanisationsbeschleunigern.

### Beispiele

Für die Bewertung der mechanischen Stabilität der Katalysatoren wurde eine schnell durchzuführende Screening-Methode entwickelt. Unter üblichen Bedingungen der hydrierenden Aminierung von Polyisobutenoxoalkoholen wurden in diskontinuierlichen Autoklavenversuchen unter standardisierten Bedingungen Umsetzungen an verschiedenen Katalysatoren durchgeführt. Die in der vorliegenden Erfindung beschriebenen Katalysatoren zeichneten sich insbesonders im Vergleich zu denen in der EP-A-382 049 beschriebenen Katalysatoren durch ihre hohe mechanische Stabilität nach Testende aus.

### Katalysatorherstellung

### Herstellung von Katalysator A

Eine wäßrige Lösung aus Nickelnitrat, Kupfernitrat und Zirkonacetat, die 4,48 % NiO, 1,52 % CuO und 2,82 % ZrO₂ enthielt, wurde gleichzeitig in einem Rührgefäß in einem konstanten Strom mit einer 20 %igen wäßrigen Natriumcarbonatlösung bei einer Temperatur von 70°C, so gefällt, daß der mit einer Glaselektrode gemessene pH-Wert von 7,0 aufrechterhalten wurde.

Die erhaltene Suspension wurde filtriert und der Filterkuchen mit vollentsalztem Wasser gewaschen bis die elektrische Leitfähigkeit des Filtrats ca. 20 µS betrug. Dann wurde in den noch feuchten Filterkuchen so viel Ammoniumheptamolybdat eingearbeitet, daß das nachfolgend angegebene Oxidgemisch erhalten wurde. Danach wurde der Filterkuchen bei einer Temperatur von 150°C in einem Trockenschrank oder einem Sprühtrockner getrocknet. Das auf diese Weise erhaltene Hydroxidcarbonatgemisch wurde nun bei einer Temperatur von 500°C über einen Zeitraum von 4 Stunden getempert.

Der so erhaltene Katalysator hatte die Zusammensetzung:

50 Gew.-% NiO, 17 Gew.-% CuO, 1,5 Gew.-% MoO₃ und 31,5 Gew.-% ZrO₂. Das Katalysatorpulver wurde mit 3 Gew.-% Graphit vermischt und zu 6 x 3 mm-Tabletten verformt. Die Tabletten hatten eine Porosität (gemessen über die Wasseraufnahme) von 0,20 ml/g und eine Härte von 3500 N/cm².

### Herstellung von Katalysator B

Für Vergleichsversuche wurde ein Kontakt anhand des EP-A-382 049 wie folgt hergestellt. Eine Lösung aus Zirkonium-, Kupfer(II)-, Kobalt(II)- und Nickel-(II)-salzen wurde simultan mit Natriumcarbonatlösung einer Dichte von 1,208 kg/l in eine Fällungsapparatur gepumpt, in der sich frisch gefälltes, in Wasser suspendiertes Zirkonium-dioxid befand. Der pH-Wert der Lösung wurde während der Fällung auf 6,0 konstant gehalten und nach Verbrauch der Mineralsalzlösung auf pH 7,5 angehoben. Der Niederschlag wurde gewaschen, bei 120°C zur Gewichtskonstanz getrocknet und bei 400°C bis zur Gewichtskonstanz calciniert. Die erhaltene Katalysatorrohmasse wurde vermahlen, mit 3 Gew.-% Graphit vermischt, tablettiert und nochmals bei 520°C 3 Stunden calciniert.

| Zusammensetzung: | |
|---|---|
| 76 Gew.% Zr, | berechnet als ZrO₂ |
| 4 Gew.% Cu, | berechnet als CuO |
| 10 Gew.% Co, | berechnet als CoO |
| 10 Gew.% Ni, | berechnet als NiO |

### Vergleichsversuch mit Katalysator A nach EP-A-382 049

Die Umsetzung wurde in einem 2 l-Autoklav durchgeführt. Der Standardhubrührer wurde mit einem 100 ml-V₂A-Behälter ausgestattet, in den jeweils 90 ml Katalysator eingefüllt wurde. Es wurde jeweils 450 g Polyisobutenoxoalkohol (50 %ige Lösung in Dodecan) mit 450 ml flüssigem Ammoniak bei 40 bar Wasserstoffdruck und 230°C und einer Reaktionszeit von 4 Stunden umgesetzt. Nach Versuchsende wurde der ausgebaute Katalysator 3 mal mit Tetrahydrofuran gewaschen, 8 h bei 125°C im Vakuum (1 mbar) getrocknet und anschließend die mechanische Stabilität bestimmt.

Der Vergleichsversuch ergab, daß die mechanische Stabilität nach Versuchsende bei Katalysator A deutlich größer ist als bei Katalysator B.

**Tabelle:**

| Vergleichsversuche zur mechanischen Stabilität | | |
|---|---|---|
| Katalysator | TYP A | TYP B |
| Seitendruckhärte vor Versuch [N] | 110,2 ± 35,2 | 39,9 ± 18,1 |
| Stirndruckhärte vor Versuch [N/cm²] | 3909 ± 1084 | 3129 ± 564 |
| Seitendruckhärte nach Versuch [N] | 40,0 ± 12,0 | 14,4 ± 8,6 |
| Stirndruckhärte nach Versuch [N/cm²] | 1835 ± 334 | 1242 ± 678 |

### Beispiel I

### Hydrierende Aminierung von Polyisobutenoxoalkohol

Ein kontinuierlich betriebener Hochdruckreaktor wurde mit 500 cm³ Katalysator A gefüllt und stündlich mit 1200 cm³ Polyisobuten-oxoalkohol (50 %ige Lösung in Dodecan) und 1200 cm³ flüssigem Ammoniak beschickt. Die Katalysatortemperatur wurde auf 210°C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert.

Die Analyse ergab folgende Werte:
Gesamt-Aminzahl: 0,54 eq/g Rohaustrag
Gesamt-Acetylierungszahl: 0,58 eq/g Rohaustrag
OH-Zahl: 0,04 eq 2,2 mg/g Rohaustrag

### Beispiel 2

### Hydrierende Aminierung von Tridecanol

Ein kontinuierlich betriebener Hochdruckreaktor wurde mit 500 cm³ Katalysator A gefüllt und stündlich mit 180 cm³ Tridecanol und 1200 cm³ flüssigem Ammoniak beschickt. Die Katalysatortemperatur wurde auf 200°C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert. Die gesammelten Reaktionsausträge wurden destilliert und gaschromatographisch analysiert:
73,8 % Tridecylamin
25,4 % Ditridecylamin
kein Tridecanol
0,7 % Rückstand

### Beispiel 3

### Hydrierende Aminierung von Diisononylphenol x 24 Butylenoxid

Ein kontinuierlich betriebener Hochdruckreaktor wurde mit 500 cm³ Katalysator A gefüllt und stündlich mit 100 cm³ Diisononylphenol x 24 Butylenoxid (Keropur ES 3213) und 300 cm³ flüssigem Ammoniak beschickt. Die Katalysatortemperatur wurde auf 220°C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert. Die Analytik der gesammelten Reaktionsausträge ergab folgende Werte:
Gesamt-Aminzahl: 0,58 eq/g Rohaustrag
Gesamt-Acetylierungszahl: 0,61 eq/g Rohaustrag
OH-Zahl: 0,03 eq/g Rohaustrag

### Beispiel 4

### Hydrierende Dimethylaminierung von Ethanol

Ein kontinuierlich betriebener Hochdruckreaktor wurde mit 500 cm³ Katalysator A gefüllt und stündlich mit 1800 cm³ eines Gemisches von Ethanol und Dimethylamin im Molverhältnis 4:1 beschickt. Die Katalysatortemperatur wurde auf 160°C und der Druck im Reaktor, durch gleichzeitiges Aufpressen von Wasserstoff, auf 60 bar eingestellt. Aus dem Reaktionsaustrag wurde nach dessen Entspannung überschüssiges Ammoniak abdestilliert. Die gesammelten Reaktionsausträge wurden gaschromatographisch analysiert:
Dimethylamin: < 0,5 %
Trimethylamin: 1,5 %
Dimethylethylamin: 24,0 %
Methyldiethylamin: 1,5 %
Ethanol: 60 %
Wasser: 6 %

### Beispiel 5

### Hydrierende Aminierung von Diglykol (Ziel: Morpholin)

Ein kontinuierlich betriebener Hochdruckreaktor wurde mit 500 cm³ Katalysator A gefüllt und stündlich mit 90 cm³ Diglykol und 350 cm³ flüssigem Ammoniak beschickt. Die Katalysatortemperatur wurde auf 200°C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert. Die gesammelten Reaktionsausträge wurden gaschromatographisch analysiert:
Morpholin: 75,8 %
Aminodiglykol: 11,8 %
Diglykol: 4,9 %
Sonstige Nebenprodukte: 7,5 %

### Beispiel 6

### Hydrierende Aminierung von Diglykol (Ziel: Aminodiglykol)

Ein kontinuierlich betriebener Hochdruckreaktor wurde mit 500 cm³ Katalysator A gefüllt und stündlich mit 270 cm³ Diglykol und 350 cm³ flüssigem Ammoniak beschickt. Die Katalysatortemperatur wurde auf 200°C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert. Die gesammelten Reaktionsausträge wurden gaschromatographisch analysiert:
Morpholin: 35,3 %
Aminodiglykol: 29,3 %
Diglykol: 30,7 %
Sonstige Nebenprodukte: 4,7 %

### Beispiel 7

### Hydrierende Aminierung von Ethylglykol

Ein kontinuierlich betriebener Hochdruckreaktor wurde mit 500 cm³ Katalysator A gefüllt und stündlich mit 150 cm³ Ethylglykol und 350 cm³ flüssigem Ammoniak beschickt. Die Katalysatortemperatur wurde auf 210°C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert. Die gesammelten Reaktionsausträge wurden gaschromatographisch analysiert:

| | |
|---|---|
| 80,7 % | Ethoxyethylamin |
| 13,9 % | Di(2-ethoxyethyl)amin |
| 3,6 % | Ethylglykol |
| 1,8 % | Sonstige Verbindungen |

### Beispiel 8

### Hydrierende Aminierung von Tripropylenglykol

Ein kontinuierlich betriebener Hochdruckreaktor wurde mit 500 cm³ Katalysator A gefüllt und stündlich mit 250 cm³ Tripropylenglykol und 1500 cm³ flüssigem Ammoniak beschickt. Die Katalysatortemperatur wurde auf 220°C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert. Die Analytik der gesammelten Reaktionsausträge ergab folgende Werte:
Gesamt-Aminzahl: 9,66 eq/g Rohaustrag
Gesamt-Acetylierungszahl: 1,02 eq/g Rohaustrag
OH-Zahl: 0,52 eq/g Rohaustrag
sek. Aminzahl: 0,61 eq/g Rohaustrag
tert. Aminzahl: 0,03 eq/g Rohaustrag

### Beispiel 9

### Hydrierende Aminierung von Polypropylenglykol

Ein kontinuierlich betriebener Hochdruckreaktor wurde mit 220 l Katalysator A gefüllt und stündlich mit 50 l Polypropylenglykol (mittlere molare Masse: 1000) und 240 l flüssigem Ammoniak beschickt. Die Katalysatortemperatur wurde auf 200°C und der Druck im Reaktor, durch gleichzeitiges Aufpressen von Wasserstoff, auf 250 bar eingestellt. Aus dem Reaktionsaustrag wurde nach dessen Entspannung überschüssiges Ammoniak abdestilliert. Die Analytik der gesammelten Reaktionsausträge ergab folgende Werte:
Gesamt-Aminzahl: 0,98 eq/g Rohaustrag
Gesamt-Acetylierungszahl: 1,00 eq/g Rohaustrag
OH-Zahl: 0,02 eq/g Rohaustrag
sek./tert. Aminzahl: 0,03 eq/g Rohaustrag

### Beispiel 10

### Hydrierende Aminierung von 2-Diisopropylethanolamin

Ein kontinuierlich betriebener Hochdruckreaktor wurde mit 500 cm³ Katalysator A gefüllt und stündlich mit 180 cm³ 2-Diisopropylethanolamin und 350 cm³ flüssigem Ammoniak beschickt. Die Katalysatortemperatur wurde auf 200°C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert. Die gesammelten Reaktionsausträge wurden gaschromatographisch analysiert:

| | |
|---|---|
| 75,7 % | N,N-Diisopropylethylendiamin |
| 1,0 % | N,N-Diisopropyl-N'-methylethylendiamin |
| 5,4 % | 2-Diisopropylethanolamin |
| 17,8 % | Sonstige Verbindungen |

## Patentansprüche

1. Verfahren zur Herstellung von Aminen aus primären oder sekundären Alkoholen und Stickstoffverbindungen, ausgewählt aus der Gruppe Ammoniak, primäre und sekundäre Amine, bei Temperaturen von 80 bis 250°C und Drücken von 1 bis 400 bar mit Wasserstoff in Gegenwart eines Zirkon-, Kupfer-, Nickelkatalysators, dadurch gekennzeichnet, daß die katalytisch aktive Masse 20 bis 85 Gew.-% sauerstoffhaltige Zirkon-Verbindungen, berechnet als ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃ und 0 bis 10 Gew.-% der sauerstoffhaltigen Verbindungen von Aluminium und/oder Mangan, berechnet als Al₂O₃ bzw. MnO₂ enthält.

2. Verfahren zur Herstellung von Aminen der allgemeinen Formel I 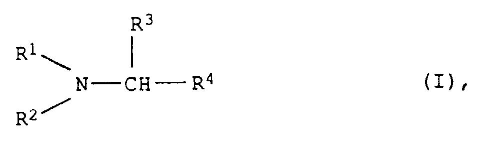 in der
R¹ und R² Wasserstoff, C₁- bis C₂₀-Alkyl, C₃- bis C₁₂-Cycloalkyl, Aryl, C₇- bis C₂₀-Aralkyl und C₇- bis C₂₀-Alkylaryl oder gemeinsam (CH₂)₁-X-(CH₂)ₘ,
R³ und R⁴ Wasserstoff, C₁- bis C₂₀₀-Alkyl, C₃- bis C₁₂-Cycloalkyl, C₁- bis C₂₀-Hydroxyalkyl, durch Amino und/oder Hydroxy substituiertes C₁- bis C₂₀-Alkyl, C₂-bis C₃₀-Alkoxyalkyl, R⁵-(OCR⁶R⁷CR⁸R⁹)ₙ-(OCR⁶R⁷), Aryl, C₇- bis C₂₀-Aralkyl, C₇- bis C₂₀-Alkylaryl, (R⁵)₂N-(CH₂)_{q} und Y-(CH₂)ₘ-NR⁵(CH₂)_{q} oder gemeinsam (CH₂)ₗ-X-(CH₂)ₘ oder
R² und R⁴ gemeinsam (CH₂)ₗ-X-(CH₂)ₘ,
R⁵ Wasserstoff, C₁- bis C₄-Alkyl, C₁₂- bis C₄₀-Alkylphenyl,
R⁶,R⁷,R⁸,R⁹ Wasserstoff, Methyl und Ethyl,
R¹⁰ Wasserstoff, C₁-C₄-Alkyl,
X CH₂, Sauerstoff oder N-R⁶,
Y N(R⁵)₂, Hydroxy, C₂- bis C₂₀-Alkylaminoalkyl oder C₃- bis C₂₀-Dialkylaminoalkyl,
n eine ganze Zahl von 1 bis 30,
l eine ganze Zahl von 2 bis 4,
m, q eine ganze Zahl von 1 bis 4
bedeuten, aus primären oder sekundären Alkoholen der allgemeinen Formel II
R⁴ - CHR³ - OH (II),
und Stickstoffverbindungen der allgemeinen Formel III 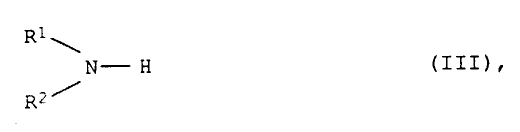 in der R¹, R² sowie R³ und R⁴ die oben genannten Bedeutungen haben, bei Temperaturen von 80 bis 250°C und Drücken von 1 bis 400 bar mit Wasserstoff in Gegenwart eines Zirkon-, Kupfer-, Nickelkatalysators, dadurch gekennzeichnet, daß die katalytisch aktive Masse 20 bis 85 Gew.-% sauerstoffhaltige Zirkon-Verbindungen, berechnet als ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃ und 0 bis 10 Gew.-% der sauerstoffhaltigen Verbindungen von Aluminium und/oder Mangan, berechnet als Al₂O₃ bzw. MnO₂ enthält.

3. Verfahren zur Herstellung von Aminen aus Alkoholen und Stickstoffverbindungen mit Wasserstoff in Gegenwart eines Zirkon-, Kupfer-, Nickelkatalysators nach Anspruch 1, dadurch gekennzeichnet, daß die katalytisch aktive Masse 40 bis 70 Gew.-% sauerstoffhaltige Verbindungen des Nickels, berechnet als NiO enthält.

4. Verfahren zur Herstellung von Aminen aus Alkoholen und Stickstoffverbindungen mit Wasserstoff in Gegenwart eines Zirkon-, Kupfer-, Nickelkatalysators nach Anspruch 1, dadurch gekennzeichnet, daß die katalytisch aktive Masse 45 bis 60 Gew.-% sauerstoffhaltige Verbindungen des Nickels, berechnet als NiO enthält.

5. Verfahren zur Herstellung von Aminen aus Alkoholen und Stickstoffverbindungen mit Wasserstoff in Gegenwart eines Zirkon-, Kupfer-, Nickelkatalysators nach Anspruch 1, dadurch gekennzeichnet, daß die katalytisch aktive Masse 0,5 bis 3,5 Gew.-% sauerstoffhaltige Verbindungen des Molybdäns, berechnet als MoO₃ enthält.

6. Verfahren zur Herstellung von Aminen aus Alkoholen und Stickstoffverbindungen mit Wasserstoff in Gegenwart eines Zirkon-, Kupfer-, Nickelkatalysators nach Anspruch 1, dadurch gekennzeichnet, daß die katalytisch aktive Masse 25 bis 60 Gew.-% sauerstoffhaltige Zirkon-Verbindungen, berechnet als ZrO₂ enthält.

7. Verfahren zur Herstellung von Aminen aus Alkoholen und Stickstoffverbindungen mit Wasserstoff in Gegenwart eines Zirkon-, Kupfer-, Nickelkatalysators nach Anspruch 1, dadurch gekennzeichnet, daß die katalytisch aktive Masse 10 bis 25 Gew.-% sauerstoffhaltige Verbindungen des Kupfers, berechnet als CuO enthält.

8. Verfahren zur Herstellung von Aminen aus Alkoholen und Stickstoffverbindungen mit Wasserstoff in Gegenwart eines Zirkon-, Kupfer-, Nickelkatalysators nach Anspruch 1, dadurch gekennzeichnet, daß man die Umsetzung bei Temperaturen von 120 bis 230°C durchführt.

9. Verfahren zur Herstellung von Aminen aus Alkoholen und Stickstoffverbindungen mit Wasserstoff in Gegenwart eines Zirkon-, Kupfer-, Nickelkatalysators nach Anspruch 1, dadurch gekennzeichnet, daß man die Umsetzung bei Drücken von 10 bis 250 bar durchführt.

10. Verfahren zur Herstellung von Aminen aus Alkoholen und Stickstoffverbindungen mit Wasserstoff in Gegenwart eines Zirkon-, Kupfer-, Nickelkatalysators nach Anspruch 1, dadurch gekennzeichnet, daß man die Umsetzung bei Drücken von 30 bis 220 bar durchführt.

## Claims

1. A process for preparing amines from primary or secondary alcohols and nitrogen compounds selected from the group consisting of ammonia and primary and secondary amines at from 80 to 250°C and pressures of from 1 to 400 bar using hydrogen in the presence of a zirconium, copper, nickel catalyst, wherein the catalytically active composition comprises from 20 to 85% by weight of oxygen-containing zirconium compounds, calculated as ZrO₂, from 1 to 30% by weight of oxygen-containing compounds of copper, calculated as CuO, from 30 to 70% by weight of oxygen-containing compounds of nickel, calculated as NiO, from 0.1 to 5% by weight of oxygen-containing compounds of molybdenum, calculated as MoO₃, and from 0 to 10% by weight of oxygen-containing compounds of aluminum and/or manganese, calculated as Al₂O₃ and MnO₂ respectively.

2. A process for preparing amines of the general formula I 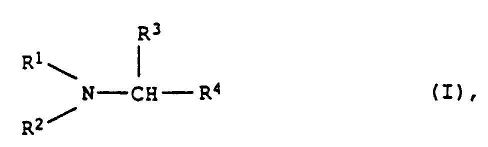 where
R¹ and R² are hydrogen, C₁- to C₂₀-alkyl, C₃- to C₁₂-cycloalkyl, aryl, C₇- to C₂₀-aralkyl and C₇- to C₂₀-alkylaryl or together (CH₂)ₗ-X-(CH₂)ₘ,
R³ and R⁴ are hydrogen, C₁- to C₂₀₀-alkyl, C₃- to C₁₂-cycloalkyl, C₁- to C₂₀-hydroxyalkyl, aminoand/or hydroxyl-substituted C₁- to C₂₀-alkyl, C₂-to C₃₀-alkoxyalkyl, R⁵-(OCR⁶R⁷CR⁸R⁹)ₙ-(OCR⁶R⁷), aryl, C₇- to C₂₀-aralkyl, C₇- to C₂₀-alkylaryl, (R⁵)₂N-(CH₂)_{q} and Y-(CH₂)ₘ-NR⁵-(CH₂)_{q} or together (CH₂)ₗ-X-(CH₂)ₘ or
R² and R⁴ are together (CH₂)ₗ-X-(CH₂)ₘ,
R⁵ is hydrogen, C₁- to C₄-alkyl, C₁₂- to C₄₀-alkylphenyl,
R⁶,R⁷,R⁸,R⁹ are hydrogen, methyl and ethyl,
R¹⁰ is hydrogen, C₁-C₄-alkyl,
X is CH₂, oxygen or N-R⁶,
Y is N(R⁵)₂, hydroxyl, C₂- to C₂₀-alkylaminoalkyl or C₃- to C₂₀-dialkylaminoalkyl,
n is an integer from 1 to 30,
l is an integer from 2 to 4,
m,q are integers from 1 to 4,
from primary or secondary alcohols of the general formula II
R⁴ - CHR³ - OH (II),
and nitrogen compounds of the general formula III 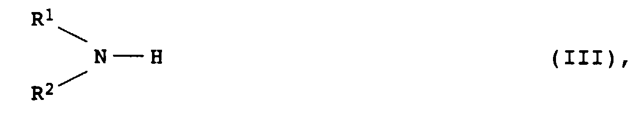 where R¹, R² and also R³ and R⁴ are as defined above, at from 80 to 250°C and pressures of from 1 to 400 bar using hydrogen in the presence of a zirconium, copper, nickel catalyst, wherein the catalytically active composition comprises from 20 to 85% by weight of oxygen-containing zirconium compounds, calculated as ZrO₂, from 1 to 30% by weight of oxygen-containing compounds of copper, calculated as CuO, from 30 to 70% by weight of oxygen-containing compounds of nickel, calculated as NiO, from 0.1 to 5% by weight of oxygen-containing compounds of molybdenum, calculated as MoO₃, and from 0 to 10% by weight of oxygen-containing compounds of aluminum and/or manganese, calculated as Al₂O₃ and MnO₂ respectively.

3. A process for preparing amines from alcohols and nitrogen compounds using hydrogen in the presence of a zirconium, copper, nickel catalyst as claimed in claim 1, wherein the catalytically active composition comprises from 40 to 70% by weight of oxygen-containing compounds of nickel, calculated as NiO.

4. A process for preparing amines from alcohols and nitrogen compounds using hydrogen in the presence of a zirconium, copper, nickel catalyst as claimed in claim 1, wherein the catalytically active composition comprises from 45 to 60% by weight of oxygen-containing compounds of nickel, calculated as NiO.

5. A process for preparing amines from alcohols and nitrogen compounds using hydrogen in the presence of a zirconium, copper, nickel catalyst as claimed in claim 1, wherein the catalytically active composition comprises from 0.5 to 3.5% by weight of oxygen-containing compounds of molybdenum, calculated as MoO₃.

6. A process for preparing amines from alcohols and nitrogen compounds using hydrogen in the presence of a zirconium, copper, nickel catalyst as claimed in claim 1, wherein the catalytically active composition comprises from 25 to 60% by weight of oxygen-containing zirconium compounds, calculated as ZrO₂.

7. A process for preparing amines from alcohols and nitrogen compounds using hydrogen in the presence of a zirconium, copper, nickel catalyst as claimed in claim 1, wherein the catalytically active composition comprises from 10 to 25% by weight of oxygen-containing compounds of copper, calculated as CuO.

8. A process for preparing amines from alcohols and nitrogen compounds using hydrogen in the presence of a zirconium, copper, nickel catalyst as claimed in claim 1, wherein the reaction is carried out at from 120 to 230°C.

9. A process for preparing amines from alcohols and nitrogen compounds using hydrogen in the presence of a zirconium, copper, nickel catalyst as claimed in claim 1, wherein the reaction is carried out at pressures of from 10 to 250 bar.

10. A process for preparing amines from alcohols and nitrogen compounds using hydrogen in the presence of a zirconium, copper, nickel catalyst as claimed in claim 1, wherein the reaction is carried out at pressures of from 30 to 220 bar.

## Revendications

1. Procédé de préparation d'amines à partir d'alcools primaires ou secondaires et de composés azotés choisis dans le groupe de l'ammoniac et des amines primaires et secondaires, à des températures de 80 à 250°C et sous des pressions de 1 à 400 bar, avec de l'hydrogène en présence d'un catalyseur au zirconium, au cuivre et au nickel, caractérisé en ce que la masse à activité catalytique contient 20 à 85% en poids de composés oxygénés du zirconium, calculés en tant que ZrO₂, 1 à 30% en poids de composés oxygénés du cuivre, calculés en tant que CuO, 30 à 70% en poids de composés oxygénés du nickel, calculés en tant que NiO, 0,1 à 5% en poids de composés oxygénés du molybdène, calculés en tant que MoO₃ et 0 à 10% en poids de composés oxygénés de l'aluminium et/ou du manganèse, calculés en tant que Al₂O₃ ou MnO₂.

2. Procédé de préparation d'amines de formule générale I 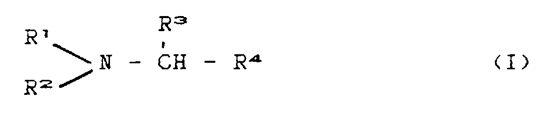 dans laquelle
R¹ et R² représentent des atomes d'hydrogène, des restes alkyle en C₁-C₂₀, cycloalkyle en C₃-C₁₂, aryle, aralkyle en C₇-C₂₀ ou alkylaryle en C₇-C₂₀, ou forment ensemble (CH₂)₁-X-(CH₂)ₘ,
R³ et R⁴ représentent des atomes d'hydrogène, des restes alkyle en C₁-C₂₀₀, cycloalkyle en C₃-C₁₂, hydroxyalkyle en C₁-C₂₀, alkyle en C₁-C₂₀ substitué par un groupement amino et/ou hydroxy, alcoxyalkyle en C₂-C₃₀, R⁵-(OCR⁶R⁷CR⁸R⁹)ₙ-(OCR⁶R⁷), aryle, aralkyle en C₇-C₂₀, alkylaryle en C₇-C₂₀, (R⁵)₂N-(CH₂)_{q} ou Y-(CH₂)ₘ-NR⁵-(CH₂)_{q}, ou forment ensemble (CH₂)₁-X-(CH₂)ₘ, ou
R² et R⁴ forment ensemble (CH₂)₁-X-(CH₂)ₘ,
R⁵ représente un atome d'hydrogène ou un reste alkyle en C₁-C₄ ou alkylphényle en C₁₂-C₄₀,
R⁶, R⁷, R⁸, R⁹ représentrent des atomes d'hydrogène ou des restes méthyle ou éthyle,
R¹⁰ représente un atome d'hydrogène ou un reste alkyle en C₁-C₄,
X représente CH₂, un atome d'oxygène ou N-R⁶,
Y représente N(R⁵)₂, un reste hydroxy, alkylaminoalkyle en C₂-C₂₀ ou dialkylaminoalkyle en C₃-C₂₀,
n est un nombre entier de 1 à 30,
l est un nombre entier de 2 à 4,
m,q sont des nombres entiers de 1 à 4,
à partir d'alcools primaires ou secondaires de formule générale II
R⁴-CHR³-OH (II)
et de composés azotés de formule générale III 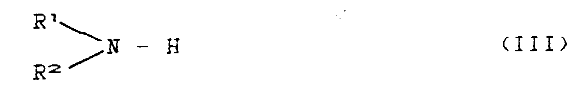 dans lesquelles R¹ et R² ainsi que R³ et R⁴ ont les significations données ci-dessus, à des températures de 80 à 250°C et sous des pressions de 1 à 400 bar, avec de l'hydrogène en présence d'un catalyseur au zirconium, au cuivre et au nickel, caractérisé en ce que la masse à activité catalytique contient 20 à 85% en poids de composés oxygénés du zirconium, calculés en tant que ZrO₂, 1 à 30% en poids de composés oxygénés du cuivre, calculés en tant que CuO, 30 à 70% en poids de composés oxygénés du nickel, calculés en tant que NiO, 0,1 à 5% en poids de composés oxygénés du molybdène, calculés en tant que MoO₃ et 0 à 10% en poids de composés oxygénés de l'aluminium et/ou du manganèse, calculés en tant que Al₂O₃ ou MnO₂.

3. Procédé de préparation d'amines à partir d'alcools et de composés azotés, avec de l'hydrogène en présence d'un catalyseur au zirconium, au cuivre et au nickel selon la revendication 1, caractérisé en ce que la masse à activité catalytique contient 40 à 70% en poids de composés oxygénés du nickel, calculés en tant que NiO.

4. Procédé de préparation d'amines à partir d'alcools et de composés azotés, avec de l'hydrogène en présence d'un catalyseur au zirconium, au cuivre et au nickel selon la revendication 1, caractérisé en ce que la masse à activité catalytique contient 45 à 60% en poids de composés oxygénés du nickel, calculés en tant que NiO.

5. Procédé de préparation d'amines à partir d'alcools et de composés azotés, avec de l'hydrogène en présence d'un catalyseur au zirconium, au cuivre et au nickel selon la revendication 1, caractérisé en ce que la masse à activité catalytique contient 0,5 à 3,5% en poids de composés oxygénés du molybdène, calculés en tant que MoO₃·

6. Procédé de préparation d'amines à partir d'alcools et de composés azotés, avec de l'hydrogène en présence d'un catalyseur au zirconium, au cuivre et au nickel selon la revendication 1, caractérisé en ce que la masse à activité catalytique contient 25 à 60% en poids de composés oxygénés du zirconium, calculés en tant que ZrO₂.

7. Procédé de préparation d'amines à partir d'alcools et de composés azotés, avec de l'hydrogène en présence d'un catalyseur au zirconium, au cuivre et au nickel selon la revendication 1, caractérisé en ce que la masse à activité catalytique contient 10 à 25% en poids de composés oxygénés du cuivre, calculés en tant que CuO.

8. Procédé de préparation d'amines à partir d'alcools et de composés azotés, avec de l'hydrogène en présence d'un catalyseur au zirconium, au cuivre et au nickel selon la revendication 1, caractérisé en ce que l'on conduit la réaction à des températures de 120 à 230°C.

9. Procédé de préparation d'amines à partir d'alcools et de composés azotés, avec de l'hydrogène en présence d'un catalyseur au zirconium, au cuivre et au nickel selon la revendication 1, caractérisé en ce que l'on conduit la réaction sous des pressions de 10 à 250 bar.

10. Procédé de préparation d'amines à partir d'alcools et de composés azotés, avec de l'hydrogène en présence d'un catalyseur au zirconium, au cuivre et au nickel selon la revendication 1, caractérisé en ce que l'on conduit la réaction sous des pressions de 30 à 220 bar.
